# NOUVEAU FASCICULE DE BREVET EUROPEEN

(11) **EP 1 284 128 B2**
(45) Date de publication et mention de la décision concernant l'opposition: **21.07.2010**
(45) Mention de la délivrance du brevet: 29.03.2006
(21) Numéro de dépôt: 02291956.7
(22) Date de dépôt: 02.08.2002
(51) Int. Cl.: A61K 8/02, A61K 8/19, A61K 8/28, A61K 8/39, A61K 8/49, A61K 8/58, A61K 8/81, A61K 8/86, A61K 8/92, A61Q 15/00

(54) **Stick antitranspirant anhydre**
Wasserfreier Antitranspirantstift
Anhydrous antiperspirant stick

(30) Priorité: 17.08.2001 FR 0110903
(43) Date de publication de la demande: 19.02.2003
(73) Titulaire: L'Oréal, 75008 Paris (FR)
(72) Inventeur: Lemoine, Cyril, 95380 Puiseux en France (FR); Douin, Véronique, 75017 Paris (FR)
(74) Mandataire: Dossmann, Gérard

(56) Documents cités:
- EP-A2- 0 135 315
- WO-A-95/30405
- WO-A1-00//10521
- CA- - 1 068 215
- DE-A1- 3 120 742
- GB- - 1 549 555
- GB-A- 1 549 555
- GB-A- 2 076 289
- US- - 4 049 792
- US- - 5 302 381
- US- - 5 378 452
- US- - 5 925 339
- US- - 6 126 928
- US-A- 5 376 363
- US-A- 5 916 546
- US-A- 6 126 928
- US-B1- 6 177 066
- COSMETICS & TOILETRIES vol. 105, Avril 1990, page 76

## Description

La présente invention a pour objet un stick antitranspirant anhydre comprenant au moins un actif antitranspirant choisi parmi le chlorhydrate d'aluminium et ses complexes notamment avec le zirconium, caractérisé par le fait qu'il comprend un agent solidifiant choisi parmi les cires d'origine naturelle ou synthétique et leurs mélanges et en outre au moins une base anhydre minérale choisie dans le groupe formé par l'hydroxyde de calcium et le carbonate de calcium.
Elle concerne également un procédé pour réduire ou supprimer le jaunissement des sticks anhydres antitranspirants à base de cires et de chlorhydrate d'aluminium ou de ses complexes, par adjonction d'une base minérale.

Les sticks antitranspirants anhydres sont connus et utilisés depuis de nombreuses années. Ils offrent de nombreux avantages par rapport à d'autres formes de présentation d'antitranspirants, en particulier par rapport aux aérosols. Ces avantages sont par exemple une grande sécurité d'utilisation et de stockage, l'absence de gaz propulseurs nocifs pour la couche d'ozone, un emballage simple et économique, une bonne stabilité à la conservation due à l'absence de phénomènes de dessèchement.

Dans le domaine cosmétique, les sticks anhydres à base de cires sont notamment bien connus dans le domaine des déodorants et antitranspirants.

On connaît dans le brevet US 5,376,363 des sticks anhydres anti-transpirants dépourvus de mono-alcool inférieur, comprenant un alcool di-hydroxylé aliphatique, comme agent gélifiant du Dibenzylidene MonoSorbitol Acetal et comme stabilisant un oxyde, hydroxyde, carbonate ou bicarbonate de métal alcalin ou alcalino-terreux ou bien un hydroxyde de métal trivalent.

Dans l'invention décrite dans la suite du texte, les sticks anhydres à base de cires sont exempts d'éthanol, d'isopropanol, de savon et de DBS (Di Benzylidène Sorbitol).

Traditionnellement on utilise une association de deux types de cires, l'une ayant un point de fusion relativement bas, à savoir inférieur à 80 °C, et l'autre ayant un point de fusion élevé c'est-à-dire supérieur à 80 °C.
Les cires à bas point de fusion sont par exemple l'alcool cétylique, l'alcool myristique, l'alcool béhénique et la plus couramment décrite dans la littérature et utilisée dans les produits commercialisés est l'alcool stéarylique qui a un point de fusion de 60 °C.

Plus récemment, dans le brevet américain N°- 6 177 066 B1, la demanderesse a décrit des sticks anhydres déodorants composés d'un mélange de cires exempt de cires à bas point de fusion, c'est-à-dire un mélange composé exclusivement de cires ayant toutes un point de fusion supérieur à 80 °C. La solidification par une telle association d'au moins deux cires à haut point de fusion, présente l'avantage pour le stick d'être plus durable à l'utilisation, de moins blanchir la peau après application, tout en conservant de bonnes propriétés à l'étalement telles que l'onctuosité et le glissant.

Cependant, la Demanderesse a pu constaté que lorsque de tels sticks anhydres à base de cires qui nécessitent donc un chauffage prolongé au delà de 60°C pour leur fabrication, contenaient comme substance antitranspirante, des chlorhydrates d'aluminium, seuls ou présents dans des complexes notamment avec le zirconium, elle obtenait des sticks jaunâtres.

Or, pour des sticks antitranspirants, l'aspect jaunâtre n'est pas du tout un critère souhaitable en terme de commercialisation.
Les consommateurs recherchent plutôt des sticks parfaitement blancs ou transparents.

C'est ainsi que la demanderesse, après de nombreuses recherches menées sur la question, a maintenant découvert, de façon inattendue et surprenante, qu'en introduisant une base minérale choisie dans le groupe formé par l'hydroxyde de calcium et le carbonate de calcium dans un stick antitranspirant à base de cires et de chlorhydrate d'aluminium ou ses complexes, on pouvait éviter leur jaunissement en fabrication sans altérer leur efficacité antitranspirante.
Ceci est d'autant plus surprenant qu'il est par ailleurs bien connu qu'en neutralisant les sels d'aluminium, on perd en efficacité antitranpirante.
En outre, on a pu constaté une bonne tenue du parfum à la chaleur et dans le temps.

Cette découverte est à la base de la présente invention.

La présente invention a ainsi pour premier objet, un stick antitranspirant essentiellement anhydre comprenant au moins un actif antitranspirant choisi dans le groupe formé par le chlorhydrate d'aluminium et ses complexes, caractérisé par le fait qu'il comprend un agent solidifiant choisi dans le groupe formé par les cires d'origine naturelle ou synthétique et leurs mélanges et en outre au moins une base anhydre minérale choisie dans le groupe formé par l'hydroxyde de calcium et le carbonate de calcium.

Elle a également pour objet, un procédé pour réduire ou supprimer le jaunissement des sticks anhydres antitranspirants à base de cires et de chlorhydrate d'aluminium ou de ses complexes, par adjonction d'une base minérale choisie dans le groupe formé par l'hydroxyde de calcium et le carbonate de calcium.

Selon la présente invention, on entend par "anhydre" que la composition du stick contient moins de 5%, de préférence moins de 3% et plus particulièrement moins de 0,5% en poids d'eau libre ou rajoutée et autre que l'eau d'hydratation associée au chlorhydrate d'aluminium, par rapport au poids total de la composition.

### Actif antitranspirant

Parmi les chlorhydrates d'aluminium et les complexes les renfermant utilisés dans les sticks antitranspirants de la présente invention, on peut citer les substances utilisées et approuvées par la F.D.A. (Food & Drug Administration) suivantes : l'aluminum chlorohydrate, l'aluminum chlorohydrex PEG, l'aluminum chlorohydrex PG, l'aluminum dichlorohydrate, l'aluminum dichlorohydrex PEG, l'aluminum dichlorohydrex PG, l'aluminum sesquichlorohydrate, l'aluminum sesquichlorohydrex PEG, l'aluminum sesquichlorohydrex PG, l'aluminum zirconium octachlorohydrate, l'aluminum zirconium octachlorohydrex GLY, l'aluminum zirconium pentachlorohydrate, l'aluminum zirconium pentachlorohydrex GLY, l'aluminum zirconium tetrachlorohydrate, l'aluminum zirconium trichlorohydrate, l'aluminum zirconium tetrachlorohydrex GLY, et l'aluminum zirconium trichlorohydrex-GLY, sachant que les sigles PEG, PG et GLY désignent respectivement polyéthylèneglycol, propylèneglycol et glycine.
Des produits commercialisés de ce type sont vendus par exemple par la société CLARIANT sous la dénomination LOCRON S (chlorhydrate d'aluminium), par la société REHEIS sous la dénomination REACH 301 ou par la société GUILINI CHEMIE sous la dénomination ALOXICOLL PF 40 (aluminum chlorohydrex), par la société REHEIS sous la dénomination REZAL 67 SOLUTION (aluminum zirconium pentachlorohydrate vendu en solution aqueuse à 40% de matière active).

Lesdits chlorhydrates d'aluminium et leurs complexes sont présents dans les sticks selon la présente invention dans des concentrations allant d'environ 1 à 50% en poids et de préférence d'environ 1 à 30% en poids de Matière Active (sur la base du sel d'aluminium anhydre à l'exclusion de l'eau et des agents complexants) par rapport au poids total de la composition.

### Bases

Lesdites bases sont présentes dans les sticks selon la présente invention dans des concentrations allant d'environ 0,01 à 20% en poids et de préférence d'environ 0,1 à 10% en poids par rapport au poids total de la composition.

### Agent solidifiant

Les cires d'origine naturelle ou synthétique et leurs mélanges qui sont utilisées dans les sticks de la présente invention pour leur conférer la consistance, renforcer leur structure et leur conférer la rigidité nécessaire à éviter leur effritement, sont choisies plus particulièrement parmi les mélanges de cires synthétiques polyéthyléniques ayant un point de fusion supérieur à 80 °C, et de cires d'origine naturelle ayant une point de fusion supérieur à 80 °C, tels qu'il est décrit dans le brevet US- 6 17706681 de la demanderesse.

On peut néanmoins utiliser des systèmes de solidification traditionnels mettant en oeuvre une ou plusieurs cires, soit d'origine naturelle, soit d'origine synthétique présentant des points de fusion pouvant être inférieurs à 80°C. Ainsi par exemple, on peut mettre en oeuvre des alcools gras ayant de 8 à 40 atomes de carbone, des alcools gras en C12-C22 éthoxylés comportant de 2 à environ 30 moles d'oxyde d'éthylène, des esters et amides d'acides gras et de mono-hydroxy et polyhydroxy-acides gras ayant de 10 à 40 atomes de carbone, des triglycérides, tous ces composés étant concrets à 25°C, des cires de silicone, d'abeille, de paraffine et d'isoparaffine. Des exemples de tels systèmes sont notamment décrits dans les demandes de brevets WO-01/13871 pages 9-10, WO-01/07007 page 29, ou WO-00/74643 pages 26-29 dont le contenu fait partie intégrante de la présente invention.

La cire polyéthylénique à haut point de fusion (> 80 °C) peut être un homopolymère d'éthylène ou un copolymère d'éthylène et d'un autre monomère copolymérisable répondant à la formule (I) suivante :

CH₂=CHR (I)

dans laquelle R représente une chaîne alkyle linéaire ou ramifiée pouvant être interrompue par des motifs mono- ou polyoxyalkylénés, un radical aryle ou aralkyle, un radical -CH₂COOH ou -CH₂CH₂OH.
Les radicaux alkyle désignent plus particulièrement les radicaux méthyle, éthyle, propyle, isopropyle, décyle, dodécyle et octadécyle.

Les motifs mono- ou polyoxyalkylénés désignent de préférence des groupes mono- ou polyoxyéthylène ou des groupes mono- ou polyoxypropylène.
Le radical aryle est de préférence un radical phényle ou tolyle.
Le radical aralkyle est par exemple un radical benzyle ou phénéthyle.
Le poids moléculaire moyen de la cire polyéthylénique à haut point de fusion est de préférence compris entre environ 400 et 1000, plus particulièrement entre environ 400 et 700 et de préférence d'environ 500.

De préférence, on la choisit parmi les homopolymères d'éthylène, les copolymères d'éthylène et de propylène, les copolymères d'éthylène et d'anhydride ou d'acide maléique, les polyéthylènes oxydés ou éthoxylés.

Parmi les homopolymères d'éthylène, on peut citer notamment ceux commercialisés sous les dénominations de POLYWAX 500, POLYWAX 655 et POLYWAX 1000 par la société PETROLITE.
Parmi les copolymères d'éthylène, on peut citer les copolymères d'éthylène et de propylène commercialisés sous les dénominations PETROLITE® par la société PETROLITE, les copolymères d'éthylène et d'anhydride maléique commercialisés sous les dénominations CERAMER® par la société PETROLITE, les polyéthylènes oxydés commercialisés sous les dénominations UNILIN® et UNICID® par la société PETROLITE, et les polyéthylènes éthoxylés commercialisés sous les dénominations UNITHOX® par la société PETROLITE.

Selon un mode de réalisation particulièrement préféré, la cire polyéthylénique est une cire d'homopolymère d'éthylène.

La cire naturelle à haut point de fusion (> 80 °C) est choisie parmi les cires minérales, fossiles, animales ou végétales, ou les huiles hydrogénées ou esters gras, les alcools gras ou alcools gras polyalcoxylés concrets à 25 °C.
A titre d'exemples de telles cires d'origine naturelle, on peut citer les cires microcristallines, la cérésine, l'ozokérite, la cire de candellila, la cire de carnauba, l'huile de ricin hydrogénée, l'huile de palme hydrogénée, l'huile de coprah hydrogénée.

Selon un mode de réalisation préféré de l'invention, la cire naturelle est une ozokérite à haut point de fusion. L'ozokérite est un hydrocarbure fossile de composition complexe correspondant au résidu solide d'évaporation de pétroles riches en paraffines.

Une ozokérite commerciale est par exemple le produit CEROZO BLANCHE E 626®, qui est un mélange d'hydrocarbures en C₂₀₋₅₀ commercialisé par la société BARLOCHER.

Généralement on mélange 5 à 20 % en poids de cire synthétique polyéthylénique et 2 à 20 % en poids de la cire naturelle à haut point de fusion (> 80 °C).
La proportion de cire polyéthylénique est de préférence supérieure à celle de la cire naturelle et le rapport pondéral (cire polyéthylénique/cire naturelle) est compris notamment entre 10/1 et 1/1 et en particulier entre 7/1 et 3/1.

Le ou les agents solidifiants sont présents dans les sticks selon la présente invention dans des concentrations allant d'environ 1 à 50% en poids et de préférence d'environ 5 à 30% en poids par rapport au poids total de la composition.

Le stick anhydre antitranspirant de l'invention peut comprendre en outre des agents émollients qui contribuent à une sensation douce, sèche, non-collante à l'application du stick sur la peau.
Ces émollients peuvent être choisis parmi des produits du type silicone volatile, des silicones non-volatiles et d'autres émollients non-volatils.

Les silicones volatiles sont définies de façon connue comme des composés volatils à température ambiante. On peut citer parmi ces composés les silicones volatiles cycliques et linéaires du type diméthylsiloxane dont les chaines comprennent de 3 à 9 résidus siliconés. De préférence on choisit les cyclométhicones telles que le cyclotétrasiloxane et le cyclopentasiloxane (D4 ou D5).
Les silicones non-volatiles sont définies de façon connue comme des composés de pression de vapeur faible à température ambiante. Parmi ces composés sont inclus : les polyalkylsiloxanes, en particulier les polyalkylsiloxanes linéaires comme par exemple les polydiméthylsiloxanes ou diméthicones, linéaires, commercialisés par la société Dow Corning sous le nom de « Dow Corning 200 Fluid » ; les polyalkylarylsiloxanes comme par exemple les polyméthylphénylsiloxanes, commercialisés par la société Dow Corning sous le nom de « Dow Corning 556 Fluid » ; les copolymères polyéther et siloxane, comme par exemple les diméthicone copolyols.
Parmi les émollients non-volatils, on peut citer par exemple, les isoparaffines tels que les polydécènes, les esters d'alcools en C₃-C₁₈ avec des acides en C₃-C₁₈, comme le monostéarate de glycérol, le monostéarate de polyéthylèneglycol, le myristate d'isopropyle, l'adipate d'isopropyle, le palmitate d'isopropyle, le palmitate d'octyle, les esters de l'acide benzoïque avec des alcools en C₁₂-C₁₈ et leurs mélanges, comme les benzoates d'alcools gras en C₁₂-C₁₅ (Finsolv TN de FINETEX), des polyols en C₂-C₆ choisis de préférence parmi le glycérol, le propylèneglycol ou le sorbitol, les polyalkylène glycol; on peut également citer le polypropylèneglycol (14) butyl éther, l'alcool myristique polyoxypropyléné à 3 moles d'oxyde de propylène (WITCONOL APM de WITCO), les triglycérides d'acides gras en C₆-C₁₈ tels que les triglycérides d'acide caprylique/caprique.
De préférence, parmi les émollients non volatils, on choisit les polydécènes, le polypropylèneglycol (14) butyl éther et le myristate d'isopropyle.

Lesdits agents émollients sont présents dans les sticks selon la présente invention dans des concentrations allant d'environ 10 à 70% en poids par rapport au poids total de la composition.

Le stick anhydre antitranspirant de l'invention peut comprendre en outre d'autres adjuvants tels que des charges comme le talc ou la silice, des adoucissants, agents structurants comme le PEG-8 distéarate, des antioxydants, des opacifiants, des stabilisants, des agents hydratants, des vitamines, des parfums, des conservateurs, ou tout autre ingrédient habituellement utilisé en cosmétique pour ce type d'application.
Bien entendu, l'homme de métier veillera à choisir ce ou ces éventuels composés complémentaires de manière telle que les propriétés avantageuses attachées intrinsèquement au stick antitranspirant conforme à l'invention ne soient pas, ou substantiellement pas, altérées par la ou les adjonctions envisagées.

Des exemples concrets, mais nullement limitatifs, illustrant l'invention vont maintenant être donnés.

### EXEMPLES 1 à 3 et exemple comparatif

On a préparé les sticks anhydres antitranspirants de composition suivante :

**(Teneurs exprimées en grammes)**

| | **Exemple 1** | **Exemple 2** | **Exemple 3** | **Exemple comparatif** |
|---|---|---|---|---|
| Cire : ozokérite........................ | 2,75 | 2,75 | 2,75 | 2,75 |
| Cire : polyéthylène (Polywax 500 de PETROLITE)...................... | 12,00 | 12,00 | 12,00 | 12,00 |
| Cyclopentasiloxane.................. | 18,90 | 18,95 | 17,40 | 19,40 |
| Polydécène..... | 15,50 | 15,75 | 14,00 | 16,00 |
| Myristate d'isopropyle............. | 15,00 | 15,00 | 15,00 | 15,00 |
| PPG-14 butyl éther.................. | 10,00 | 10,00 | 10,00 | 10,00 |
| PEG-8 distéarate...................... | 2,50 | 2,50 | 2,50 | 2,50 |
| Antioxydant................................. | 0,05 | 0,05 | 0,05 | 0,05 |
| Parfum........................................ | 1,30 | 1,30 | 1,30 | 1,30 |
| Base : Carbonate de calcium..... | 2,00 | | 5,00 | |
| Base : Hydroxyde de calcium..... | | 0,70 | | |
| Charge: Talc.................................... | | 1,00 | | 1,00 |
| Aluminium Zirconium Tetrachlorohydrex Gly................... | 20,00 | 20,00 | 20,00 | 20,00 |
| | 100,00 | 100,00 | 100,00 | 100,00 |

Les cires, agents émollients (polydécène, myristate d'isopropyle, PPG-14 butyl éther) et structurant (PEG-8 distéarate), base, charge et conservateur ont été mélangés et chauffés à une température d'environ 60 à 90 °C et mélangés jusqu'à ce que les cires aient été fondues et que le mélange soit devenu homogène. On a ensuite ajouté lentement le cyctopentasiloxane tout en maintenant la température entre 60 et 90°C. On a ensuite ajouté l'Aluminium Zirconium Tetrachlorohydrex Gly à la même température. Au mélange ensuite refroidi vers 65°C, on a ajouté le parfum.

Sur chacun des sticks obtenus, on a effectué des mesures colorimétriques au colorimètre MINOLTA CM2002 dans le système L* a* b*
Dans ce système, la mesure du paramètre b*, désigne plus la valeur de b* est élevée, plus la nuance est jaune.
On a obtenu une valeur de b* d'environ 1,3 pur les sticks des exemples 1, 2, et 3 selon l'invention et une valeur d'environ 6,3 pour le stick de l'exemple comparatif sans base et hors invention.

Par ailleurs, les sticks des exemples 1 à 3 selon l'invention ont présenté la même efficacité antitranspirante que celle de l'exemple comparatif hors invention.

### EXEMPLE 4 et exemple comparatif

On a préparé les sticks anhydres antitranspirants de composition suivante :

**(Teneurs exprimées en grammes)**

| | **Exemple 4** | **Exemple comparatif** |
|---|---|---|
| Alcool stéarylique..... | 14,00 | 14,00 |
| Huile de ricin hydrogénée........................... | 4,00 | 4,00 |
| Talc...................................................... | 3,20 | 3,20 |
| PEG-8 distéarate..... | 1,00 | 1,00 |
| Hydroxyde de calcium..... | 2,00 | |
| Cyclopentasiloxane................................... | 50,80 | 52,80 |
| Parfum.................................................... | 1,00 | 1,00 |
| Aluminium Zirconium Tetrachlorohydrex Gly | 24,00 | 24,00 |
| | 100,00 | 100,00 |

On a procédé comme dans les exemples précédents, sauf que la température de fusion a été de 65 à 75°C.
Le stick de l'exemple 4 selon l'invention a donné une valeur de b* moins élevée que celle de l'exemple comparatif hors invention et son efficacité antitranspirante n'a pas diminué par rapport à celle de l'exemple comparatif.

## Revendications

1. Stick antitranspirant anhydre comprenant au moins un actif antitranspirant choisi dans le groupe formé par le chlorhydrate d'aluminium et ses complexes et moins de 5% en poids d'eau libre ou rajoutée et autre que l'eau d'hydratation associée au chlorhydrate d'aluminium par rapport au poids total de la composition, **caractérisé par le fait qu'**il comprend un agent solidifiant choisi dans le groupe formé par les cires d'origine naturelle ou synthétique et leurs mélanges et en outre au moins une base anhydre minérale choisie dans le groupe formé par l'hydroxyde de calcium et le carbonate de calcium ;
le stick étant exempt d'éthanol, d'isopropanol, de savon et de dibenzylidène sorbitol.

2. Stick selon la revendication 1, **caractérisé par le fait que** l'actif antitranspirant est le chlorhydrate d'aluminium et ses complexes avec le zirconium.

3. Stick selon l'une quelconque des revendications précédentes, **caractérisé par le fait que** les cires d'origine naturelle sont choisies dans le groupe formé par les cires minérales, fossiles, animales ou végétales, ou les huiles hydrogénées, les esters gras, les alcools gras et les alcools gras polyalcoxylés concrets à 25 °C.

4. Stick selon la revendication 3, **caractérisé par le fait que** les cires d'origine naturelle sont choisies dans le groupe formé par une cire microcristalline, cérésine, ozokérite, cire de candelilla, cire de carnauba, huile de ricin hydrogénée, huile de palme hydrogénée, huile de coprah hydrogénée, des triglycérides, une cire d'abeille, de paraffine et d'isoparaffine.

5. Stick selon l'une quelconque des revendications 1 ou 2, **caractérisé par le fait que** les cires d'origine synthétique sont choisies dans le groupe formé par les cires polyéthyléniques et les cires de silicone.

6. Stick selon la revendication 5, **caractérisé par le fait que** la cire polyéthylénique est un homopolymère de l'éthylène dont le poids moléculaire est de 500 approximativement.

7. Stick selon l'une quelconque des revendications précédentes, **caractérisé par le fait que** l'agent solidifiant est un mélange de cire naturelle et de cire polyéthylénique ayant chacune un point de fusion supérieur à 80 °C.

8. Stick selon la revendication 7, **caractérisé par le fait que** le rapport pondéral entre la cire polyéthylénique et la cire naturelle est compris entre 10/1 et 1/1 et de préférence entre 7/1 et 3/1.

9. Stick selon l'une quelconque des revendications précédentes, **caractérisé par le fait que** l'actif antitranspirant représente de 1 à 50 % de Matière Active en poids par rapport au poids total de la composition et de préférence de 1 à 30%.

10. Stick selon l'une quelconque des revendications précédentes, **caractérisé par le fait que** la base représente de 0,01 à 20% de Matière Active en poids par rapport au poids total de la composition et de préférence de 0,1 à 10%.

11. Stick selon l'une quelconque des revendications précédentes, **caractérisé par le fait que** l'agent solidifiant représente de 1 à 50 % de Matière Active en poids par rapport au poids total de la composition et de préférence de 5 à 30%.

12. Stick selon l'une quelconque des revendications précédentes, **caractérisé par le fait qu'**il comprend en outre un ou plusieurs agents émollients.

13. Stick selon la revendication 12, **caractérisé par le fait que** les agents émollients sont choisis dans le groupe formé par les silicones volatiles, les polydécènes, le polypropylèneglycol (14) butyl éther, le myristate d'isopropyle.

14. Stick selon la revendication 13, **caractérisé par le fait que** les agents émollients représentent de 10 à 70% de Matière Active en poids par rapport au poids total de la composition.

15. Stick selon l'une quelconque des revendications précédentes, **caractérisé par le fait qu'**il comprend en outre une ou plusieurs charges et autres adjuvants.

16. Procédé pour réduire ou supprimer le jaunissement des sticks anhydres antitranspirants à base de cires et de chlorhydrate d'aluminium ou de ses complexes, par adjonction d'une base minérale choisie dans le groupe formé par l'hydroxyde de calcium et le carbonate de calcium.

## Claims

1. Anhydrous antiperspirant stick comprising at least one antiperspirant active agent chosen from the group consisting of aluminium chlorohydrate and its complexes and less than 5% by weight of free or added water and other than the water of hydration combined with the aluminium chlorohydrate relative to the total weight of the composition, **characterized in that** it comprises a solidifying agent chosen from the group consisting of waxes of natural or synthetic origin and mixtures thereof and, in addition, at least one inorganic anhydrous base chosen from the group consisting of calcium hydroxide and calcium carbonate, the stick being free of ethanol, isopropanol, soap and dibenzylidene sorbitol.

2. Stick according to Claim 1, **characterized in that** the antiperspirant active agent is aluminium chlorohydrate and its complexes with zirconium.

3. Stick according to any one of the preceding claims, **characterized in that** the waxes of natural origin are chosen from the group consisting of mineral, fossil, animal or plant waxes, or hydrogenated oils, fatty esters, fatty alcohols and polyalkoxylated fatty alcohols which are concrete at 25°C.

4. Stick according to Claim 3, **characterized in that** the waxes of natural origin are chosen from the group consisting of a microcrystalline wax, ceresin, ozokerite, candelilla wax, carnauba wax, hydrogenated castor oil, hydrogenated palm oil, hydrogenated copra oil, triglycerides, beeswax, paraffin and isoparaffin.

5. Stick according to any one of Claims 1 or 2, **characterized in that** the waxes of synthetic origin are chosen from the group consisting of polyethylene waxes and silicone waxes.

6. Stick according to Claim 5, **characterized in that** the polyethylene wax is a homopolymer of ethylene whose molecular weight is approximately 500.

7. Stick according to any one of the preceding claims, **characterized in that** the solidifying agent is a mixture of natural wax and polyethylene wax each having a melting point greater than 80°C.

8. Stick according to Claim 7, **characterized in that** the weight ratio between the polyethylene wax and the natural wax is between 10/1 and 1/1 and preferably between 7/1 and 3/1.

9. Stick according to any one of the preceding claims, **characterized in that** the antiperspirant active agent represents from 1 to 50% of Active Substance by weight relative to the total weight of the composition, and preferably from 1 to 30%.

10. Stick according to any one of the preceding claims, **characterized in that** the base represents from 0.01 to 20% of Active Substance by weight relative to the total weight of the composition, and preferably from 0.1 to 10%.

11. Stick according to any one of the preceding claims, **characterized in that** the solidifying agent represents from 1 to 50% of Active Substance by weight relative to the total weight of the composition, and preferably from 5 to 30%.

12. Stick according to any one of the preceding claims, **characterized in that** it comprises, in addition, one or more emollients.

13. Stick according to Claim 12, **characterized in that** the emollients are chosen from the group consisting of volatile silicones, polydecenes, polypropylene glycol (14)butyl ether, isopropyl myristate.

14. Stick according to Claim 13, **characterized in that** the emollients represent from 10 to 70% of Active Substance by weight relative to the total weight of the composition.

15. Stick according to any one of the preceding claims, **characterized in that** it comprises, in addition, one or more fillers and other adjuvants.

16. Method for reducing or eliminating the yellowing of anhydrous antiperspirant sticks based on waxes and on aluminium chlorohydrate or its complexes, by the addition of an inorganic or base chosen from the group consisting of calcium hydroxide and calcium carbonate.

## Patentansprüche

1. Wasserfreier Antitranspirantstift, der mindestens ein Antihidrotikum, das unter Aluminiumhydroxychlorid und seinen Komplexen ausgewählt ist, und weniger als 5 Gew.-% freies oder hinzugefügtes Wasser, das von den mit dem Aluminiumhydroxychlorid kombinierten Hydratwasser verschieden ist, bezogen auf das Gesamtgewicht der Zusammensetzung, enthält, **dadurch gekennzeichnet, dass** es ein Verfestigungsmittel, das unter den Wachsen natürlicher oder synthetischer Herkunft und deren Gemischen ausgewählt ist, und ferner mindestens eine wasserfreie anorganische Base enthält, die unter Calciumhydroxyd und Calciumcarbonat ausgewählt ist, wobei der Stift kein Ethanol, kein Isopropanol, keine Seife und kein Dibenzylidensorbit enthält.

2. Stift nach Anspruch 1, **dadurch gekennzeichnet, dass** es sich bei dem Antihidrotikum um das Aluminiumhydroxychlorid uns seine Komplexe mit Zirkonium handelt.

3. Stift nach einem der vorhergehenden Ansprüche, **dadurch gekennzeichnet, dass** die Wachse natürlicher Herkunft unter den mineralischen, fossilen, tierischen oder pflanzlichen Wachsen oder hydrierten Ölen, Fettsäureestern, Fettalkoholen und alkoxylierten Fettalkoholen ausgewählt sind, die bei 25°C fest sind.

4. Stift nach Anspruch 3, **dadurch gekennzeichnet, dass** die Wachse natürlicher Herkunft unter mikrokristallinen Wachsen, Ceresin, Ozokerit, Candelillawachs, Carnaubawachs, hydriertem Ricinusöl, hydriertem Palmöl, hydriertem Kopraöl, Triglyceriden, Bienenwachs, Paraffinwachs und Isoparaffinwachs ausgewählt sind.

5. Stift nach einem der Ansprüche 1 oder 2, **dadurch gekennzeichnet, dass** die Wachse synthetischer Herkunft unter den Polyethylenwachsen und Siliconwachsen ausgewählt sind.

6. Stift nach Anspruch 5, **dadurch gekennzeichnet, dass** das Polyethylenwachs ein Homopolymer von Ethylen ist, dessen Molmasse etwa 500 beträgt.

7. Stift nach einem der vorhergehenden Ansprüche, **dadurch gekennzeichnet, dass** das Verfestigungsmittel ein Gemisch aus einem natürlichen Wachs und einem Polyethylenwachs ist, die beide einem Schmelzpunkt über 80°C besitzen.

8. Stift nach Anspruch 7, **dadurch gekennzeichnet, dass** das Gewichtsverhältnis von Polyethylenwachs und natürlichem Wachs im Bereich von 10/1 bis 1/1 und vorzugsweise 7/1 bis 3/1 liegt.

9. Stift nach einem der vorhergehenden Ansprüche, **dadurch gekennzeichnet, dass** das Antihidrotikum in einer Menge von 1 bis 50% wirksame Substanz, bezogen auf das Gesamtgewicht der Zusammensetzung, und vorzugsweise 1 bis 30 Gew.-% vorliegt.

10. Stift nach einem der vorhergehenden Ansprüche, **dadurch gekennzeichnet, dass** die Base als wirksame Substanz 0,01 bis 20 Gew.-%, bezogen auf das Gesamtgewicht der Zusammensetzung, und vorzugsweise 0,1 bis 10 Gew.-% ausmacht.

11. Stift nach einem der vorhergehenden Ansprüche, **dadurch gekennzeichnet, dass** das Verfestigungsmittel als wirksame Substanz 1 bis 50 Gew.-%, bezogen auf das Gesamtgewicht der Zusammensetzung, und vorzugsweise 5 bis 30 Gew.-% ausmacht.

12. Stift nach einem der vorhergehenden Ansprüche, **dadurch gekennzeichnet, dass** er ferner ein oder mehr Emollientien enthält.

13. Stift nach Anspruch 12, **dadurch gekennzeichnet, dass** die Emollientien unter den flüchtigen Silikonen, Polydecenen, Polypropylenglykol(14)butylether und Isopropylmyristat ausgewählt sind.

14. Stift nach Anspruch 13, **dadurch gekennzeichnet, dass** die Emollienten als wirksame Substanz 10 bis 70 Gew.-%, bezogen auf das Gesamtgewicht der Zusammensetzung, ausmachen.

15. Stift nach einem der vorhergehenden Ansprüche, **dadurch gekennzeichnet, dass** er ferner ein oder mehrere Füllstoffe und weitere Zusatzstoffe enthält.

16. Verfahren zur Verminderung oder Verhinderung des Gelbwerdens von wasserfreien Antitranspirantstiften auf der Basis von Wachsen und Aluminiumhydroxychlorid oder seinen Komplexen durch Zugabe einer anorganischen Base, die unter Calciumhydroxyd und Calciumcarbonat ausgewählt ist.
